# EUROPÄISCHE PATENTSCHRIFT

(11) **EP 0 942 713 B1**
(45) Veröffentlichungstag und Bekanntmachung des Hinweises auf die Patenterteilung: **12.03.2003**
(21) Anmeldenummer: 97951960.0
(22) Anmeldetag: 24.11.1997
(51) Int. Cl.: A61K 7/50

(54) **SCHÄUMENDE KÖRPERREINIGUNGSMITTEL**
FOAMING BODY-CLEANSING AGENTS
AGENTS MOUSSANTS POUR LE NETTOYAGE DU CORPS

(30) Priorität: 02.12.1996 DE 19649895
(43) Veröffentlichungstag der Anmeldung: 22.09.1999
(73) Patentinhaber: Henkel Kommanditgesellschaft auf Aktien, 40191 Düsseldorf (DE)
(72) Erfinder: SCHELGES, Heike, D-47807 Krefeld (DE); SCHOLZ, Wolfhard, D-47829 Krefeld (DE); SCHOSSER, Gryta, D-47829 Krefeld (DE)
(86) Internationale Anmeldenummer: EP9706556
(87) Internationale Veröffentlichungsnummer: WO98024409

(56) Entgegenhaltungen:
- DE-A- 4 131 992
- DE-A- 4 234 487
- DE-A- 4 301 820
- DE-A- 4 435 387

## Beschreibung

Die Erfindung betrifft schäumende Körperreinigungsmittel in Form einer flüssigen Tensidzusammensetzung auf Basis einer Kombination stark schäumender Aniontenside und schaumverstärkender Alkyl-(poly)-glycoside, die zur weiteren Verbesserung der Schäumeigenschaften, insbesondere der Feinblasigkeit und Beständigkeit (Cremigkeit) des Schaums eine Kombination aus einem zwitterionischen Tensid und einem ampholytischen Tensid enthalten.

Flüssige Körperreinigungsmittel, wie sie z.B. als flüssige Seifen, Shampoos, Duschbad-Zubereitungen und Schaumbad-Zusätze im Handel sind, müssen nicht nur ein gutes Reinigungsvermögen aufweisen, sondern sollen für Haut und Schleimhäute gut verträglich sein und auch bei häufiger Anwendung nicht zu starker Entfettung oder Hauttrockenheit fuhren. Darüber hinaus beurteilt der Verbraucher die Gebrauchseigenschaften aber auch nach der Menge und Qualität des bei der Anwendung sich bildenden Schaumes. Insbesondere wird ein rasches Anschäumen unter Bildung von feinblasigem und beständigem Schaum gewünscht, wobei diese Eigenschaften des Schaums auch meist als die Cremigkeit des Schaums beschrieben werden.

Auch die Körperreinigungsmittel selbst sollen sich durch eine gewisse Dickflüssigkeit auszeichnen, so daß sie z.B. auf die Hand aufgebracht werden können und nicht, bevor sie auf der Körperoberfläche oder auf dem Kopf verteilt werden können, zwischen den Fingern hindurchrinnen.

Es sind zahlreiche hautfreundliche und schleimhautverträgliche Tenside bekannt. Die zusätzliche Forderung nach einer gewissen Dickflüssigkeit der wäßrigen Lösung und nach einer Feinblasigkeit des Schaums wird jedoch nur von wenigen Tensiden erfüllt. Man hat daher auch bisher stets Kombinationen verschiedener Tenside verwendet, um den vielseitigen Anforderungen gerecht zu werden. So hat sich eine Kombination aus Alkylethersulfat-Tensiden und Alkyl-(poly)-glucosiden als besonders schaumstark und hautverträglich erwiesen. Weiterhin sind zwitterionische Tenside bzw. Betaintenside und ampholytische Tenside dafür bekannt, daß sie in Kombination mit anionischen Tensiden deren Hautverträglichkeit verbessern und den wäßrigen Zubereitungen eine höhere Viskosität verleihen oder ihrer Verdickbarkeit durch Elektrolytsalze verbessern.

Aus DE-A-42 34 487 ist z.B. eine wäßrige Detergenszusammensetzung bekannt, die Alkylsulfat-Tenside, Alkylethersulfat-Tenside, Alkyl-(oligo)-glucoside und amphotere bzw. zwitterionische Tenside enthält.

Aus DE-A-44 35 387 ist eine wäßrige Detergenszusammensetzung bekannt, die Alkyloligoglykoside, Sulfosuccinate und amphotere bzw. zwitterionische Tenside enthält.

Es wurde nun aber festgestellt, daß sich die Schäumeigenschaften, insbesondere die Feinblasigkeit und Beständigkeit des Schaumes weiter steigern lassen, wenn man anstelle eines Betain- oder Amphotensids eine Kombination aus einem zwitterionischen und einem ampholytischen Tensid einsetzt.

Gegenstand der Erfindung sind daher wäßrige Körperreinigungsmittel-Zusammensetzungen mit einem Gehalt an schaumstarken, hautverträglichen anionischen Tensiden (A) und Alkyl-(oligo)-glycosiden (B), die dadurch gekennzeichnet sind, daß sie zur weiteren Verbesserung der Schaumeigenschaften und der Viskosität eine Kombination aus einem zwitterionischen Tensid (C) der Formel I 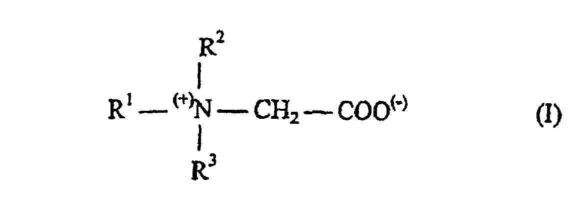 und einem Amphotensid (D) der Formel II 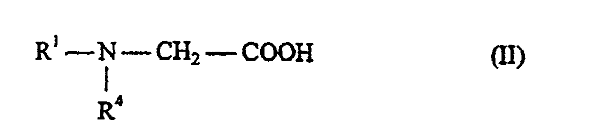 worin R¹ eine Alkyl- oder Alkenylgruppe mit 12 - 18 C-Atomen oder eine Gruppe R⁵ ― CONH ― (CH₂)ₙ― ist, worin R⁵ eine Alkyl- oder Alkenylgruppe mit 12 - 18 C-Atomen und n eine Zahl von 2 - 4 ist und R², R³ und R⁴ Alkylgruppen mit 1 - 4 C-Atomen oder Hydroxyalkylgruppen mit 2 oder 3 C-Atomen sind, in einem Gewichtsverhältnis von (A) : (B) : (C + D) = 10 : (0,5 - 5) : (1 - 5) enthalten.

Schaumstarke, hautverträgliche anionische Tenside (A) sind dem Fachmann in großer Zahl aus einschlägigen Handbüchern bekannt und im Handel erhältlich. Es handelt sich dabei insbesondere um Alkylsulfate in Form ihrer Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 - 18 C. Atomen und in Form ihrer Alkali- oder Ammoniumsalze. Die anionischen Tenside (A) können dabei in einer Menge von 3 - 30 Gew.-% in den erfindungsgemäßen Zusammensetzungen enthalten sein. Besonders bevorzugt eignen sich als anionische Tenside die Alkylethersulfate; es ist daher bevorzugt als anionisches Tensid wenigstens ein Alkylethersulfattensid der Formel III

R⁶O(C₂H₄O)ₘ - SO₃⁽⁻⁾ M⁽⁺⁾ (III)

in der R⁶ eine Alkyl- oder Alkenylgruppe mit 12 - 18 C-Atomen, m = 1 - 4 und M⁽⁺⁾ ein Alkali-, Magnesium-, Ammonium- oder Alkanolammoniumion ist, in einer Menge von wenigstens 5 Gew.-% der gesamten Zusammensetzung enthalten.

Alkyl-(oligo)-glycoside (B) sind seit langem bekannte, oberflächenaktive Stoffe, die aus Zuckern und aliphatischen, primären Alkoholen mit 8 - 22 C-Atomen unter Acetalisierung herstellbar sind. Als Zuckerkomponenten (Glycosen) kommen bevorzugt Glucose, daneben aber auch Fructose, Mannose, Galactose, Talose, Gulose, Allose, Idose, Arabinose, Xylose, Lyxose, Ribose und Gemische davon in Frage. Bevorzugt wegen der leichten Verfügbarkeit und der guten Anwendungseigenschaften sind die Acetalisierungsprodukte der Glucose mit Fettalkoholen, die z.B. aus natürlichen Ölen und Fetten nach bekannten Verfahren erhältlich sind.

Bezüglich des Glycosidrestes gilt, daß sowohl Monoglycoside als auch Oligoglycoside, bei denen ein Zuckerrest glycosidisch an den Fettalkohol gebunden ist, geeignet sind. Gewöhnlich liegen bei den Handelsprodukten Gemische von Mono- und Oligoglucosiden vor.

Bevorzugt sind als Alkyl-(oligo)-glycoside (B) solche der Formel R⁷(G)ₓ enthalten, worin R⁷ eine lineare Alkylgruppe mit 8 - 16 C-Atomen und (G)ₓ ein (Oligo)-Glucosidrest mit einem mittleren Oligomerisationsgrad x von 1 bis 2 ist

Solche Produkte sind z.B. unter dem Warenzeichen Plantaren® oder Plantacare® im Handel.

Zwitterionische Tenside (C) der Formel I sind ebenfalls seit langem bekannt und in einer Vielzahl im Handel erhältlich. Die bekannteste und am weitesten verbreitete Gruppe dieser Tenside ist die der Betain-Tenside, bei denen R² und R³ Methylgruppen sind.

Weiterhin unterscheidet man dabei die Alkylbetaine, bei denen R¹ eine Alkyl- oder Alkylengruppe ist und die Amidbetaine, bei welchen R¹ eine Gruppe R⁵CONH-(CH₂)ₙ- ist. Bevorzugt geeignet für die Zwecke der Erfindung ist ein Cocoamidopropylbetain der Formel I, in der R¹ einen Rest R⁵CONH(CH₂)₃- darstellt, worin R⁵CO von einer C₁₂ - C₁₈-Kokos- oder Palmkernfsttsäure abgeleitet ist und R² und R³ Methylgruppen sind. Solche Produkte sind z.B. unter dem Warenzeichen Dehyton®K im Handel.

Amphotenside (D) der Formel II sind ebenfalls bekannte und im Handel erhältliche Tenside. Sie haben die Eigenschaft, in saurer Lösung durch Protonierung am tertiären Stickstoffatom wie Kationtenside und im alkalischen Bereich durch Salzbildung an der Carboxylgruppe wie anionische Tenside zu reagieren. Bevorzugt geeignet als ampholytisches Tensid (D) ist ein Cocoamphoglycinat der Formel II, in der R¹ ein Rest R⁵CONH(CH₂)₂-, worin R⁵CO von einer C₁₂-C₁₈-Kokos- oder Palmkernölfettsäure abgeleitet ist und R⁴ eine Hydroxyethylgruppe ist Ein solches Tensid ist z.B. unter dem Warenzeichen Dehyton®G im Handel erhältlich.

Besonders günstige Schäumeigenschaften, insbesondere eine gute Feinblasigkeit und Cremigkeit des Schaums wird erzielt, wenn das zwitterionische Tensid (C) und das Amphotensid (D) im Gewichtsverhältnis (C): (D) = 1 : (0,1 0,5) enthalten sind.

Die erfindungsgemäßen schäumenden, wäßrigen Zubereitungen können neben den obligatorischen Komponenten (A), (B), (C) und (D) auch noch weitere Tenside und Zusätze enthalten. Solche weiteren Stoffe sollten in ihrer Menge insgesamt nicht mehr als die Komponente (A) ausmachen.

Geeignete weitere Zusätze sind z.B. nichtionogene Tenside, wasserlösliche Polymere, z.B. kationische polymere Avivagemittel, perlglanzbildende Stoffe, Farbstoffe, Duftstoffe und dafür geeignete Emulgatoren, wasserlösliche Polyole wie z.B. Glycerin, Sorbit, Propylenglycol oder Polyethylenglycol, Elektrolytsalze, pH-Stellmittel und kosmetische oder dermatologische Wirkstoffe.

Wenn der Gehalt der Komponenten (A) und (B) insgesamt unter 10 Gew.-% liegt, kann die Viskosität erfindungsgemäßer Körperreinigungsmittel noch unbefriedigend sein. Es ist ein besonderer Vorteil der erfindungsgemäßen Zubereitungen, daß sich in solchen Fällen die Viskosität leicht durch den Zusatz wasserlöslicher anorganischer Elektrolytsalze anheben läßt.

Als anorganische Elektrolytsalze eignen sich alle wasserlöslichen Alkali-, Ammoniumund Erdalkalisalze, z.B. die Fluoride, Chloride, Bromide, Sulfate, Phosphate, Nitrate und Hydrogencarbonate, soweit sie in einer Menge von wenigstens 1 Gew.-% bei 20°C in Wasser löslich sind. Bevorzugt werden Natriumchlorid und Magnesiumchlorid verwendet

Die erfindungsgemäßen Körperreinigungsmittel-Zusammensetzungen können als hochkonzentrierte Pasten mit einem Wassergehalt von weniger als 30 Gew.-% H₂O oder als verdünnte wäßrige Lotionen mit weniger als 5 Gew.-% des anionischen Tensids (A) formuliert werden. Bevorzugt liegt aber der Gehalt an anionischen Tensiden (A) im Bereich von 5 - 20 Gew.-%. In diesem Bereich lassen sich erfindungsgemäße Körperreinigungsmittel in einem Viskositätsbereich von ca. 1 bis 200 Pa·s (20° C) formulieren, die sich als Shampoos oder Duschgele eignen.

Die folgenden Beispiele sollen den Erfindungsgegenstand näher erläutern:

### Beispiele

### I. Eingesetzte Tenside

- Texapon®N70:: C_{12/14}-Kokosfettalkohol-2 EO-Addukt-Sulfat
-Na-Salz (70 %ige Paste)
- Dehyton®K:: Cocoamidopropyl-betain (30 %ige Lösung) 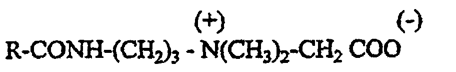 R¹CO = Acylgruppe der C₈-C₁₈-Kokosfettsäure
- Dehyton®G:: Cocoamphocarboxyglycinate (30 %ige Lösung)
R²-CONH(CH₂)₂ - NH (CH₂CH₂OH) CH₂-COOH
R²CO = Acylgruppe der C₈-C₁₈-Kokosfettsäure
- Plantacare®818:: C₈-C₁₄-Alkylpolyglucosid (50 %ige Lösung)
R³-(G)ₓ, R³ = C₈-C₁₄-n-Alkylgruppe
x = 1,4 G = Glucosidrest
- Cetiol® HE:: PEG7-Glyceryl-cocoate
- Euperlan®PK 810:: Perlglanzkonzentrat, enthält Glycoldistearat und
Texapon N70.

### II. Beurteilung der Schaummenge und Qualität

Schäumvermögen und Schaumeigenschaften wurden in einem praxisnahen standardisierten Armwaschtest geprüft. 2 g der Zusammensetzung wurden auf die nasse Hand gegeben und die Hände und Unterarme damit eingeseift. Nach 1 Minute wurde mit Wasser (15° C) abgespült. Das Ausschäumverhalten, die Schaummenge und Cremigkeit des Schaums wurden benotet. Die Beurteilung von zehn Prüfpersonen wurde gemittelt 1 = mangelhaft, 2 = ausreichend, 3 = gut, 4 = sehr gut

| **Rezepturen** | | | | | | |
|---|---|---|---|---|---|---|
| **Gew.-%** | **1** | **2V** | **3V** | **4V** | **5V** | **6V** |
| Texapon N 70 | 15 | 15 | 15 | 17 | 18 | 16 |
| Plantaren 818 | 2 | 2 | 2 | 4 | - | - |
| Dehyton K | 6 | 8 | - | - | - | 6 |
| Dehyton G | 2 | - | 8 | - | 2 | 2 |
| Euperlan PK 810 | 2 | 2 | 2 | 2 | 2 | 2 |
| Cetiol HE | 0,5 | 0,5 | 0,5 | 0,5 | 0,5 | 0,5 |
| Merquat 550 | 0,5 | 0,5 | 0,5 | 0,5 | 0,5 | 0,5 |
| Sorbit | 1,5 | 1,5 | 1,5 | 1,5 | 1,5 | 1,5 |
| Glycerin | 1,0 | 1,0 | 1,0 | 1,0 | 1,0 | 1,0 |
| Natriumbenzoat | 0,4 | 0,4 | 0,4 | 0,4 | 0,4 | 0,4 |
| Milchsäure | 0,1 | 0,1 | 0,1 | 0,1 | 0,1 | 0,1 |
| Citronensäure | 0,4 | 0,4 | 0,4 | 0,4 | 0,4 | 0,4 |
| Parfümöl | 1,3 | 1,3 | 1,3 | 1,3 | 1,3 | 1,3 |
| Wasser | 67,3 | 67,3 | 67,3 | 71,3 | 72,3 | 68,3 |
| NaCl | 0,2 | 0,2 | 0,2 | 0,2 | 0,2 | 0,2 |
| Anschäumverhalten | 2,9 | 2,1 | 1,8 | 2,0 | 2,0 | 1,9 |
| Schaummenge | 3,0 | 2,3 | 2,0 | 2,0 | 2,0 | 1,9 |
| Cremigkeit, Feinblasigkeit | 3,5 | 2,2 | 2,5 | 2,1 | 2,1 | 2,1 |
| Viskosität Pa.s (20° C) Haake Rotovisko, Spindel 2 | 9,5 | 4,0 | 15,4 | < 1,0 | < 1,0 | 6,1 |

## Patentansprüche

1. Schäumende, wäßrige Körperreinigungsmittel-Zusammensetzung mit einem Gehalt an schaumstarken, hautverträglichen anionischen Tensiden (A) und Alkyl-(oligo)-glycosiden (B), **dadurch gekennzeichnet, daß** zur Verbesserung der Schaumeigenschaften und der Viskosität eine Kombination aus einem zwitterionischen Tensid (C) der Formel I 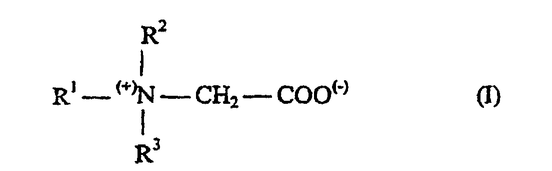 und einem Amphotensid (D) der Formel II 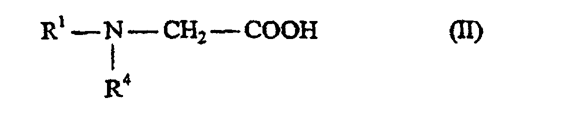 worin R¹ eine Alkyl- oder Alkenylgruppe mit 12-18 C-Atomen oder eine Gruppe R⁵ - CO - NH(CH₂)ₙ - ist, worin R⁵ eine Alkyl- oder Alkenylgruppe mit 12 - 18 C-Atomen und n eine Zahl von 2 - 4 ist, und R², R³ und R⁴ Alkylgruppen mit 1 -4 C-Atomen oder Hydroxyalkylgruppen mit 2 oder 3 C-Atomen sind, in einem Gewichtsverhältnis von (A) : (B) : (C + D) = 10 : (0,5 - 5) : (1 - 5) enthalten ist.

2. Schäumende Körperremigungsmittel-Zusammensetzung nach Anspruch 1, **dadurch gekennzeichnet, daß** als anionisches Tensid wenigstens ein Alkylethersulfat-Tensid der Formel III
R⁶O (C₂H₄O)ₘ - SO₃⁽⁻⁾ M⁽⁺⁾ (III)
in der R⁶ eine Alkyl- oder Alkenylgruppe mit 12 - 18 C-Atomen, m = 1 bis 4 und M⁽⁺⁾ ein Alkali-, Magnesium-, Ammonium- oder Alkanolammonium-Ion ist. in einer Menge von wenigstens 5 Gew.-% enthalten ist

3. Schäumende Körperreinigungsmittel-Zusammensetzung nach Anspruch 1, **dadurch gekennzeichnet, daß** als Alkyl-(oligo)-glycosid ein Alkylglucosid der Formel R⁷(G)ₓ enthalten ist, worin R⁷ eine lineare Alkylgruppe mit 8 - 16 C-Atomen und (G)ₓ ein (Oligo)-Glucosidrest mit einem Oligomerisationsgrad x von 1 bis 2 ist.

4. Schäumende Körperreinigungsmittel-Zusammensetzung nach einem der Ansprüche 1 bis 3, **dadurch gekennzeichnet, daß** als zwitterionisches Tensid (C) ein Cocoamidopropyl-Betain der Formel I enthalten ist, in der R¹ ein Rest R⁵CONH(CH₂)₃- ist, worin R⁵CO von einer C₁₂-C₁₈-Kokos- oder Palmkemfettsäure abgeleitet ist, und R² und R³ Methylgruppen sind.

5. Schäumende Körperreinigungsmittel-Zusammensetzung nach einem der Ansprüche 1 bis 4, **dadurch gekennzeichnet, daß** als Amphotensid (D) ein Cocoamphoglycinat der Formel II enthalten ist, in der R¹ ein Rest R⁵CONH(CH₂)₂ - ist, worin R⁵CO von einer C₁₂-C₁₈-Kokos- oder Palmkernfettsäure abgeleitet ist und R⁴ eine Hydroxyethylgruppe ist

6. Schäumende Körperreinigungsmittel-Zusammensetzung nach einem der Ansprüche 1 bis 5, **dadurch gekennzeichnet, daß** das zwitterionische Tensid (C) und das Amphotensid (D) im Gewichtsverhältnis (C) : (D) = 1 : (0,1 - 0,5) enthalten sind.

## Claims

1. A foaming aqueous body-cleansing composition containing high-foaming, dermatologically compatible anionic surfactants (A) and alkyl (oligo)glycosides (B), **characterized in that** it contains a combination of a zwitterionic surfactant (C) corresponding to formula I: 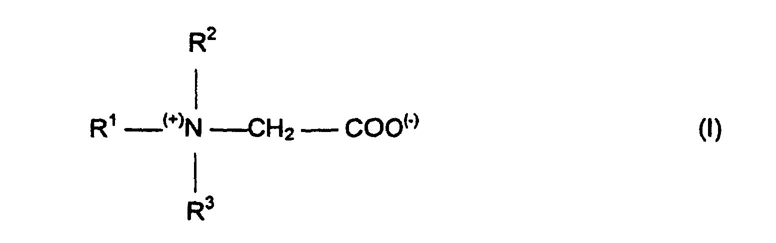 and an ampholytic surfactant (D) corresponding to formula II: 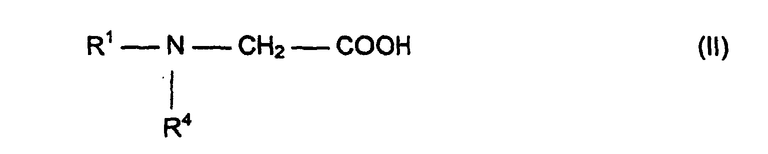 in which R¹ is an alkyl or alkenyl group containing 12 to 18 carbon atoms or a group R⁵ ― CONH ― (CH₂)ₙ, where R⁵ is an alkyl or alkenyl group containing 12 to 18 carbon atoms and n is a number of 2 to 4, and R², R³ and R⁴ are alkyl groups containing 1 to 4 carbon atoms or hydroxyalkyl groups containing 2 or 3 carbon atoms,
in a ratio by weight of (A) to (B) to (C + D) of 10:(0.5-5):(1-5)
in order further to improve its foam properties and its viscosity.

2. A foaming body-cleansing composition as claimed in claim 1, **characterized in that** at least one alkyl ether sulfate surfactant corresponding to formula III:
R⁶O (C₂H₄O)ₘ - SO₃⁽⁻⁾ M⁽⁺⁾ (III)
in which R⁶ is an alkyl or alkenyl group containing 12 to 18 carbon atoms, m = 1 - 4 and M⁽⁺⁾ is an alkali metal, magnesium, ammonium or alkanolammonium ion,
is present as the anionic surfactant in a quantity of at least 5% by weight.

3. A foaming body-cleansing composition as claimed in claim 1, **characterized in that** an alkyl glucoside with the formula R⁷(G)ₓ, where R⁷ is a linear alkyl group containing 8 to 16 carbon atoms and (G)ₓ is an (oligo)glucoside unit with a degree of oligomerization x of 1 to 2, is present as the alkyl (oligo)glycoside.

4. A foaming body-cleansing composition as claimed in any of claims 1 to 3, **characterized in that** a cocoamidopropyl betaine corresponding to formula I, in which R¹ is a group R⁵CONH-(CH₂)₃-, in which R⁵CO is derived from a C₁₂₋₁₈ cocofatty acid or palm kernel oil fatty acid, and R² and R³ are methyl groups is present as the zwitterionic surfactant (C).

5. A foaming body-cleansing composition as claimed in any of claims 1 to 4, **characterized in that** a cocoamphoglycinate corresponding to formula II where R¹ is a group R⁵CONH-(CH₂)₂-, in which R⁵CO is derived from a C₁₂₋₁₈ cocofatty acid or palm kernel oil fatty acid, and R⁴ is a hydroxy ethyl group is present as the ampholytic surfactant (D).

6. A foaming body-cleansing composition as claimed in any of claims 1 to 5, **characterized in that** the zwitterionic surfactant (C) and the ampholytic surfactant (D) are present in a ratio by weight of (C) to (D) of 1:(0.1-0.5).

## Revendications

1. Composition aqueuse d'agents moussants pour le nettoyage du corps contenant des tensioactifs anioniques tolérés par la peau et fortement moussants (A) et des alkyl-(oligo)-glycosides (B), **caractérisée en ce que** pour améliorer les propriétés moussantes et la viscosité, elle contient une association d'un tensioactif zwitterionique (C) de la formule I 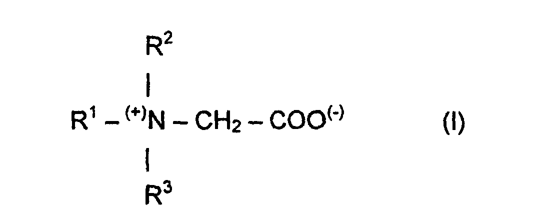 et d'un tensioactif amphotère (D) de la formule II 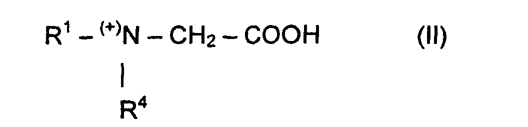 dans laquelle R¹ représente un groupe alkyle ou alcényle comportant 12 à 18 atomes de C ou un groupe R⁵ - CO - NH(CH₂)ₙ , dans lequel R⁵ correspond à un groupe alkyle ou alcényle possédant 12 à 18 atomes de C et n est un nombre de 2 à 4, R², R³ et R⁴ représentent des groupes alkyle possédant 1 à 4 atomes de C ou des groupes hydroxyalkyle comportant 2 ou 3 atomes de C, dans un rapport pondéral de (A) : (B) : (C + D) = 10 : (0,5 - 5) : (1 - 5).

2. Composition d'agents moussants pour le nettoyage du corps selon la revendication 1, **caractérisée en ce que** comme tensioactif anionique, elle contient, dans une proportion d'au moins 5 % en poids, au moins un tensioactif d'alkyléthersulfate de la formule IIII
R⁶O (C₂H₄O)ₘ - SO₃⁽⁻⁾ M⁽⁺⁾ (III)
dans laquelle R⁶ représente un groupe alkyle ou alcényle comportant 12 à 18 atomes de C, m = 1 à 4 et M⁽⁺⁾ correspond à un ion de métal alcalin, de magnésium, d'ammonium ou d'alcanolammonium.

3. Composition d'agents moussants pour le nettoyage du corps selon la revendication 1, **caractérisée en ce que** comme alkyl-(oligo)-glycoside, elle contient un alkyglucoside de la formule R⁷(G)ₓ, dans laquelle R⁷ représente un groupe alkyle linéaire comportant 8 à 16 atomes de C et (G)ₓ est un radical (oligo)-glucoside présentant un degré d'oligomérisation x de 1 à 2.

4. Composition d'agents moussants pour le nettoyage du corps selon une des revendications 1 à 3, **caractérisé en ce que** comme surfactif zwitterionique (C), elle contient une cocoamidopropyl-bétaïne de la formule I, dans laquelle R¹ représente un radical R⁵CONH(CH₂)₃, dans lequel R⁵CO est dérivé d'un acide gras de coco ou de palmiste en C₁₂-C₁₈ et R² et R³ sont des groupes méthyle.

5. Composition d'agents moussants pour le nettoyage du corps selon une des revendications 1 à 4, **caractérisé en ce que** comme tensioactif amphotère (D), elle contient un cocoamphoglycinate de la formule II, dans laquelle R¹ représente un radical R⁵CONH(CH₂)₂, dans lequel R⁵CO est dérivé d'un acide gras de coco ou de palmiste en C₁₂-C₁₈ et R⁴ est un groupe hydroxyéthyle.

6. Composition d'agents moussants pour le nettoyage du corps selon une des revendications 1 à 5, **caractérisé en ce que** le surfactif zwitterionique (C) et le tensioactif amphothère (D) sont entre eux dans un rapport pondéral (C) : (D) = 1 : (0,1 - 0,5).
